# EUROPEAN PATENT SPECIFICATION

(11) **EP 3 034 682 B1**
(45) Date of publication and mention of the grant of the patent: **23.08.2017**
(21) Application number: 15201313.2
(22) Date of filing: 18.12.2015
(51) Int. Cl.: D06F 58/20, D06F 58/28

(54) **FABRIC TREATING APPARATUS**
GEWEBEBEHANDLUNGSVORRICHTUNG
APPAREIL DE TRAITEMENT DE TISSU

(30) Priority: 19.12.2014 KR 20140184787
(43) Date of publication of application: 22.06.2016
(73) Proprietor: LG Electronics Inc., Seoul 07336 (KR)
(72) Inventor: AHN, Jaehyun, Seoul 08592 (KR); DOH, Youngjin, Seoul 08592 (KR); KIM, Sunyong, Seoul 08592 (KR)
(74) Representative: Vossius & Partner Patentanwälte Rechtsanwälte mbB

(56) References cited:
- EP-A1- 0 821 096
- EP-A1- 2 514 863
- EP-A1- 2 514 865
- DE-U1-202012 103 881
- US-A1- 2008 155 757
- US-A1- 2010 218 566

## Description

The present invention relates to a fabric treating apparatus.

A fabric treating apparatus refers to any device which is used at home or at a laundry to manage clothes by washing, drying, and flatten out the same.

For example, the fabric treating apparatus includes a washing machine, a drying machine, a washer-and-dryer, a refresher, a steamer for removing creases on fabric, and the like.

The refresher is designed to keep clothes in a clean and fresh condition, and, for this purpose, it sprays aroma to the fabric, prevents static electricity of the fabric, removes creases on the fabric, and the like.

A general iron removes creases on fabric simply by providing steam thereto. On the other hand, the steamer removes creases without heating the clothes.

A fabric treating apparatus having the functions of the refresher and the steamer is able to remove creases and unpleasant smell of fabric by using steam and heated air.

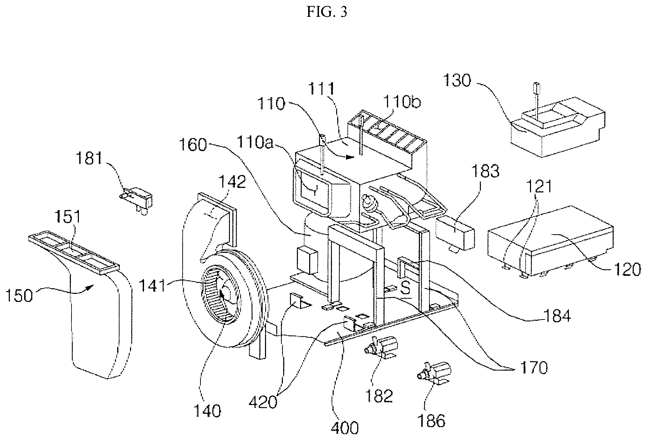

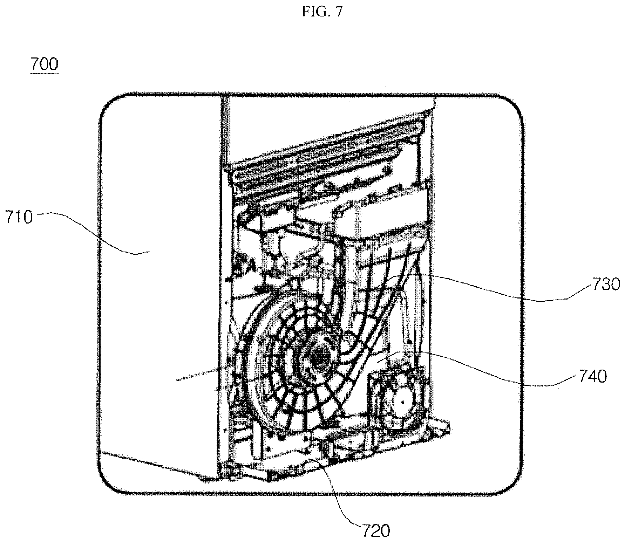

FIG. 7 is a diagram illustrating a machinery room of a conventional fabric treating apparatus. Referring to FIG. 7, a conventional fabric treating apparatus 700 has a machinery room below a casing 710, wherein various devices are installed in the machinery room. The devices to be installed in the machinery room include not only a blower 730 and a compressor 740 which are shown in FIG. 7, but also a steam generating module, a heat pump module, and a controller which are shown in FIG. 3. Among them, the steam generating module, the heat pump module and/or the controller are not able to be drawn out from the machinery room for maintenance or repair without removing other devices, because the steam generating module, the heat pump module and/or the controller interfere with the blower 730, the compressor 740, and the like, as shown in FIG. 7. Accordingly, in a conventional method, a device in need of repair is removed after devices causing interference are removed, or a base 720 itself is detached from the casing 10 with all the devices being installed in the machinery room. However, in this method, maintaining and repairing the devices is cumbersome and requires much time.

EP 2 514 865 A1 discloses an apparatus according to the preamble of claim 1.

The present invention aims to provide a fabric treating apparatus in which arrangement of devices in a machinery room is improved, thereby making it easy to maintain and repair the devices.

In addition, the present invention aims to provide a fabric treating apparatus which solves a problem that it is necessary to draw out other devices from a machinery room to draw out a specific device.

Further, the present invention aims to provide a fabric treating apparatus of which a controller, a steam generating module, and a heat pump module are capable of being independently drawn out from a machinery room.

Moreover, the present invention aims to provide a fabric treating device with improved space utilization of a machinery room.

In an aspect, there is provided a fabric treating apparatus comprising: a casing including a treating chamber in which clothes are kept, and a machinery room disposed below the treating chamber; a base defining a bottom of the machinery room; a heat pump module performing air-conditioning on air supplied to the treating chamber; at least one supporter supporting the heat pump module and limiting a predetermined space between the at least one supporter and the base below the heat pump module; a steam generating module fixed onto the space and generating steam to be supplied to the treating chamber; and a controller controlling at least one of the heat pump module and the steam generating module, the controller disposed below the steam generating module in the space. The supporter comprises a pair of legs fixed on the base; and a shelf supported by the legs, wherein the heat pump module is supported by the shelf.

A supporter fixing tab including a surface facing the base may be formed at a lower part of the leg, and a supporter fixing part including a supporter fastening hole into which the supporter fixing tab is inserted may be formed in the base. The base may be made of a metal material, and the supporter fixing part may be formed by plastic working of the base. The supporter fixing part may be formed by cutting a portion having a predetermined length from the base in a direction crossing a direction in which the supporter fixing tab is inserted and then upwardly pressing a predetermined peripheral area of the cut portion. The supporter fastening hole may be formed due to plastic deformation of the cut portion.

The base and the supporter may be coupled to each other by a fastening member which penetrates the supporter fixing part and the supporter fixing tab in a vertical direction.

The casing may include an opening formed at a rear side of the controller to allow the controller to pass therethrough, the heat pump module may be disposed above the controller, and the fabric treating apparatus may further comprise a blower which transfers air into an interior of the heat pump module. The fabric treating apparatus may further comprise a panel detachably coupled to the casing and closing the opening of the machinery room. The blower may be disposed in front of the supporter. The blower may be coupled to the base.

The fabric treating apparatus may further comprise an air intake duct guiding air flow introduced into the blower, wherein the air intake duct may be disposed in front of the blower. The air intake duct may be fixed by at least one of the blower and the base.

A steam generating module fixing tab may be formed at one of the steam generating module and the supporter, and an installation hole into which the steam generating module fixing tab is inserted may be formed at the other. The pair of supporters may be spaced apart from each other in a front and rear direction of the base, the steam generating module installation part may be formed on one of the pair of the supporters, and the steam generating module may be coupled to the other one of the pair of the supporters by a fastening member while the steam generating module fixing tab is inserted into the installation hole.
FIG. 1 is a perspective view illustrating a fabric treating apparatus according to an exemplary embodiment of the present disclosure.
FIG. 2 is a diagram illustrating a device assembly installed in a machinery room shown in FIG. 1.
FIG. 3 is a exploded perspective illustrating the device assembly shown in FIG. 2.
FIG. 4 shows a procedure of how to assemble the device assembly shown in FIG. 2.
FIG. 5 is a diagram illustrating a structure in which a supporter is mounted on a base.
FIG. 6 is a diagram illustrating an installation structure of a heat pump module.
FIG. 7 is a diagram illustrating a machinery room of a conventional fabric treating apparatus.

Hereinafter, detailed descriptions related to well-known functions or configurations will be ruled out in order not to unnecessarily obscure subject matters of the present invention. Although the same term is used to indicate different elements, different numeric references may be used for the elements.

Also, terms used in this specification are terms defined in consideration of functions according to embodiments, and thus the terms may be changed according to the intention or usage of a user or operator. Therefore, the terms should be defined on the basis of the overall contents of this specification.

In the present disclosure, terms such as "first" and "second" may be used to explain various elements, but the elements are not limited to such terms. The terms are used only to distinguish one element from other elements. For example, within the scope of the claims of the present disclosure, the first element may be named the second element, and similarly the second element may be named the first element. The term "and/or" includes a combination of plural items or any one of the plural items.

A term used in the present disclosure is used to explain a specific exemplary embodiment, not intended to limit the present disclosure. Any term in the singular may be interpreted in the plural as long as such interpretation coincides with a context.

Every term including a technical or scientific term has an identical meaning that is generally understood by those skilled in the art if it is not differently defined. The general terms, such as a term defined in the dictionary, should be interpreted as the meaning according to a contextual meaning of the related technology, and should not be interpreted as an ideal or excessively formal meaning as long as they are not obviously defined in the present invention.

Further, the terms 'include', 'construct', or 'have' used in the above description have a meaning of including the corresponding constructional element as long as there is no specific contrary description. Therefore, it should be interpreted as further including another constructional element, not to exclude another constructional element.

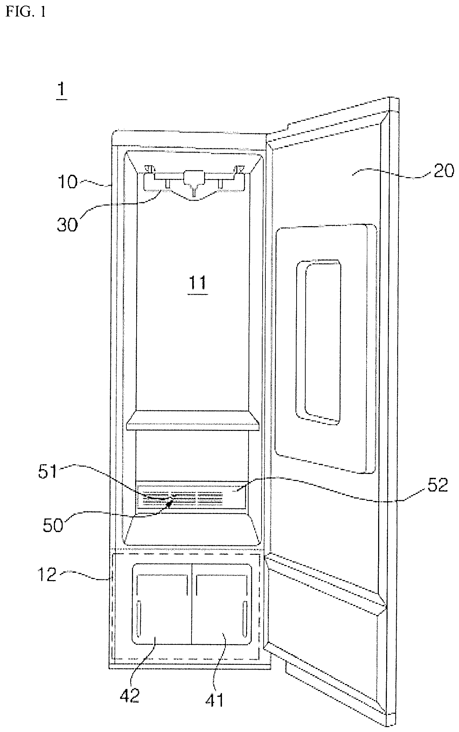

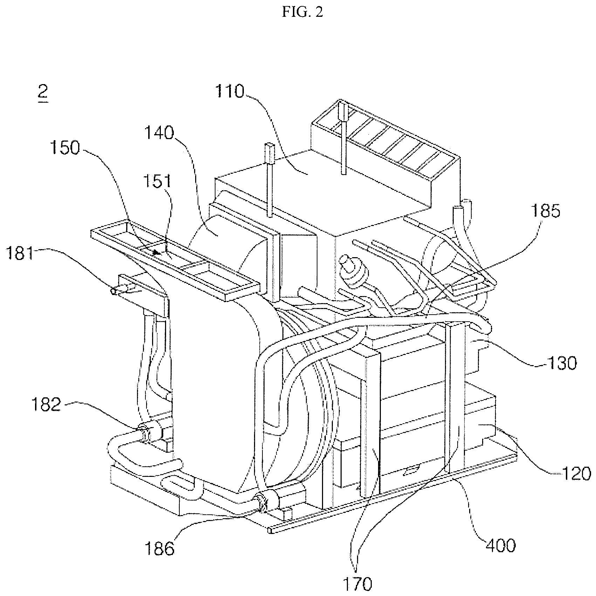

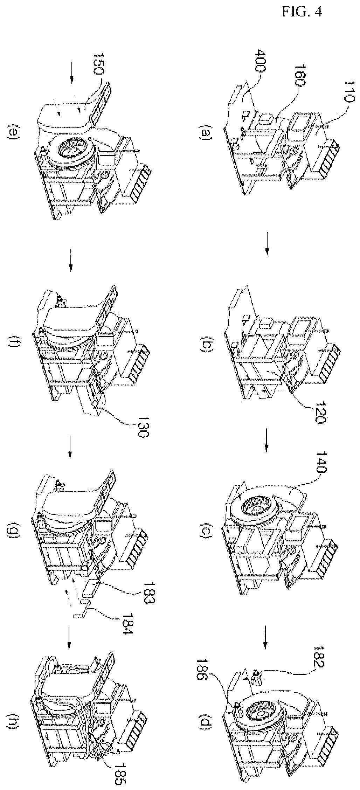

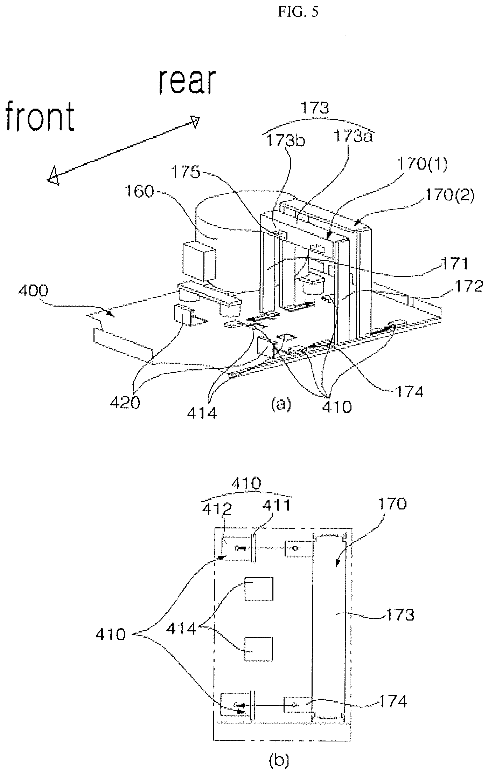

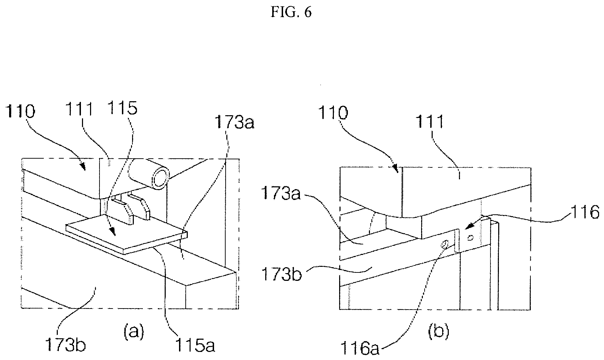

FIG. 1 is a perspective view of a fabric treating apparatus according to an exemplary embodiment of the present disclosure. FIG. 2 is a device assembly installed in a machinery room shown in FIG. 1. FIG. 3 is a exploded perspective view of the device assembly shown in FIG. 2. FIG. 4 is a diagram illustrating a procedure of how to assemble the device assembly shown in FIG. 2. FIG. 5 is a diagram illustrating a base having a supporter mounted therein. FIG. 6 is a diagram illustrating an installation structure of a heat pump module.

Referring to FIGS. 1 to 6, a fabric treating apparatus 1 according to an exemplary embodiment of the present disclosure includes a casing 10 forming an open front treating chamber 11, and a door for closing the open front of the treating chamber 11.

A hanger rod 30 on which clothes hangers are to be hung is provided in an upper part of the treating chamber 11. The hanger rod 30 may be configured to move in a back and forth, up and down and/or right and left direction in the treating chamber 11 by a driving device (not shown), such as a motor. Preferably, such movement is a reciprocating motion with a predetermined cycle.

A machinery room 12 may be provided outside the treating chamber 11 (preferably, below the treating chamber 11) within the casing 10, and various devices, such as a heat pump module 110, a controller 120, and a steam generating module 130, may be disposed in the machinery room 12. In particular, the casing 10 may include a base 400 which defines a bottom of the machinery room 12. The base 400 may be formed with a metal sheet.

An air outlet 51 discharging air blown by a blower 140 into the treating chamber 11, and a steam outlet 52 discharging steam generated by a stream generating module 130 may be formed in the lower part of the treating chamber 11. In this exemplary embodiment, the air outlet 51 and the steam outlet 52 are formed as one body on a low panel 50 which defines a lower part of the treating chamber 11, but not limited thereto.

The steam generating module 130 is a device which generates steam. The steam generating module 130 may include a heater for applying heat to water. Steam generated according to an operation of the heater may be guided to the steam outlet 52 via a predetermined steam supply path (not shown).

A water supply tank 41 for storing water to be supplied to the steam generating module 130 may be disposed in the lower part of the treating chamber 11. The water supply tank 41 may be detachably coupled to the casing 10. A user may fill the water supply tank 41 with water after detaching the water supply tank 41 from the casing 10.

A sump (not shown) may be disposed in the machinery room 12 to collect condensed water which is generated while steam generated by the steam generating module 130 moves along the steam supply path. The sump may be connected to the treating chamber 11 via a path, in this case, the condensed water on the bottom of the treating chamber 11 may be gathered in the sump.

A water drain pump 182 may be mounted on a base 400 to discharge the condensed water from the sump to a drain container 42. The drain container 42 may be detachably coupled to the casing 10. A user may detach the drain container 42 from the casing 10 and empty the drain container 42. The sump may be disposed in an inner low part of an air intake duct 150, and a nozzle module 181 may be further provided to discharge the condensed water stored in the sump to the drain container 42 via the water drain pump 182.

On the base 400, a water supply pump 186 may be mounted to supply water stored in the water supply tank 41 to the steam generating module 130. The water supply pump 186 may be connected to the steam generating module 130 via a water supply hose 185.

The heat pump module 110 is provided for air conditioning. The conditioned air is supplied to the treating chamber 11. The heat pump module 110 may be connected to the compressor 160 via a refrigerant line. The heat pump module 110 may include a condenser, an expansion valve, and an evaporator through which refrigerant passes in order when transferred via the refrigerant line.

Referring to FIG. 3, the heat pump module 110 may include a housing 111 forming an inner space where the condenser is housed. An air inlet 110a and an air outlet 110b may be formed in the housing 111. The air inlet 110a introduces air into the space where the condenser is housed, and the air outlet 110b discharges air being heat exchanged with the condenser. Not only the condenser, but also the evaporator may be housed in the housing 111.

The blower 140 is an air blowing device, and may include a fan which is rotated by a motor. A vent 142 discharging air blown by the fan is connected to the air inlet 110a of the housing 111. Accordingly, the air blown by the blower 140 is supplied to the housing 111 through the air inlet 110a, and the supplied air is heated by heat exchanging with the condenser. And the heated air is discharged through the air outlet 110b of the housing 111. In this embodiment, the blower 140 is a centrifugal blower configured to guide the air, which is introduced through an opening 141 opened in an axial direction of the fan (e.g. centrifugal fan), toward the vent 142 via a scroll-type path embracing the surroundings of the fan. However, aspects of the present disclosure are not limited thereto.

The air intake duct 150 guiding air toward the opening 141 of the blower 140 may be disposed in front of the blower 140. The air intake duct 150 may be fixed by the blower 140. However, not limited thereto, the duct 150 may be fixed by the base 400.

A blower installation tab 420 may be formed in the base 400. The blower installation tab 420 may formed by cutting a portion of the base 400 and bending the cut portion in a vertical direction. The blower 140 may be coupled to the blower installation tab 420 by a fastening member, such as a bolt and a screw.

The air intake duct 150 may include an air inflow path through which air passes. For efficient use of the inner space of the machinery room 12, the air intake duct 150 may be extended upward from the opening 141 of the blower 140. The air inflow path may have a width in a front-and-rear direction, which is smaller than a width in a left-and-right direction. The air intake duct 150 may have an opening 151 formed on a top surface thereof, and an outlet (not shown) connected the vent 142 of the blower 140. Air inside the treating chamber 11 may be introduced through the opening 151.

The supporter 170 supports the heat pump module 110, and limits a predetermined space S below the heat pump module 110. The space S is formed between the support 170 and the base 400 below the heat pump module 110. That is, as being supported by the supporter 170, the heat pump module 110 is disposed in an upper side being spaced apart from the base 400. The heat pump module 110 is spaced apart from the base 400, thereby the space S is provided in the base 400.
Devices, for example the steam generating module 130 and the controller 120, are mounted in the space S.

A plurality of supporters 170 may be provided. In this embodiment, a pair of supporters 170 are spaced apart from each other in a front-and-rear direction on the base 400. The pair of supporters 170 may include supporter 170(1), 170(2). The supporter 170(1) may locate further forward than the supporter 170(2).

Referring to FIG. 5, the supporter 170 may include at least two legs 171 and 172 which are fixed on the base 400, and a shelf 173 which is extended in a lateral direction and supported by the legs 171 and 172. A supporter fixing tab 174 having a surface facing the base 400 may be formed at a lower part of the legs 171 and 172. As shown in (a) of FIG. 5, a supporter fixing part 410 may be formed in the base 400. The supporter fixing part 410 has a fastening hole 411 into which the supporter fixing tab 174 is inserted in a front-and-rear direction of the fabric treating apparatus 1.

The supporter fixing part 410 may be formed by cutting a portion having a predetermined length from the base 400 in a direction crossing a direction in which the supporter fixing tab 174 is inserted and then upwardly pressing a predetermined peripheral area 412 of the cut portion (that is, the supporter fastening hole 411).

With the supporter fixing tab 174 being inserted into the supporter fixing part 410 through the supporter fastening hole 411, an upward movement of the supporter 170 is restrained as the supporter fixing tab 174 is retained by a bottom surface of the peripheral area 412 of the supporter fastening hole 411.

At least two supporters 170 may be provided. In this case, as shown in FIG. 5, as the supporter 170(1) disposed in front part of the base 400 moves forward, the supporter fixing tab 174 may be inserted into the supporter fastening hole 411. As the supporter 170(2) disposed in rear part of the base 400 moves backward, the supporter fixing tab 400 may be inserted into the supporter fixing part 410 which is open toward the front direction. The base 400 and the supporter 170 may be coupled to each other by a fastening member, such as a screw or a bolt which penetrates the supporter fixing part 410 and the supporter fixing tab 174 in an up-and-down direction.

The supporter 170 may be formed of a plastically deformable metal material. And the supporter fixing part may be formed by plastic working of the base.

By cutting and bending a metal plate according to a predetermined lay out, the supporter 170 may be formed to have the legs 171 and 172, the supporter fixing tab 174, and the shelf 173, which are integrated in the supporter 170 as one body. However, aspects of the present disclosure are not limited thereto. Thus, the supporter 170 may be formed of a molded product of synthetic resin, or may be formed with two or more components coupled to one another.

The heat pump module 110 may be disposed on the shelf 173. The shelf 173 may include a pair of side surfaces 173b which faces front side and rear side, respectively, and a top surface 173a connecting the pair of side faces 173b. The shelf 173 may be formed by bending a metal plate at least twice.

Referring to FIG. 6, at least one connecting part 115 and 116 to be coupled to the shelf 173 may be formed in a lower part of the housing 111. Referring to (a) of FIG. 6, the connecting part 115 may include a first contact surface 115a which contacts the top surface 173a of the shelf 173. The first contact surface 115a is substantially parallel with the top surface 173a. After the first contact surface 115a is put on the top surface 173a of the shelf 173, the first contact surface 115a and the top surface 173a of the shelf 173 may be coupled to each other by a fastening member.

Referring to (b) of FIG. 6, the connecting part 116 may have a second contact surface 116a which contacts the side surface 173b of the shelf 173. In this case, the second contact surface 116a is vertically disposed corresponding to the side surface 173b, and may be coupled to the side surface 173b of the shelf 173 by a fastening member. Preferably, the first contact surface 115a is secured to the top surface 173a of the supporter 170(1), and the second contact surface 116a is secured to the side surface 173b of the supporter 170(2).

The controller 120 controls various devices composing the fabric treating apparatus 1, and may include a circuit substrate and electric components mounted on the circuit substrate. The heat pump module 110, the steam generating module 130, the blower 140 and/or the compressor 160 may be controlled by the controller 120.

The circuit substrate is accommodated in a body which defines an exterior of the controller 120. The controller 120 may be disposed in the space S between the supporter 170 and the base 400. Preferably, the controller 120 is disposed on the base 400.

Referring to FIGS. 3, 4, and 5, a controller fixing tab 121 may be formed in a body of the controller 120, and a controller fixing hole 414 into which the controller fixing tab 121 is inserted may be formed on the base 400. The controller fixing tab 121 may be a plate which is extended forward or backward. With the controller fixing tab 121 being inserted into the controller fixing hole 414, upward movement of the controller 120 may be restrained because the controller fixing tab 121 is retained by a bottom surface of the base 400.

Preferably, the controller fixing tab 121 projects in a forward direction from the body of the controller 120, and may be inserted into or separated from the controller fixing hole 414 according to forward or backward movement of the controller 120 on the base 400.

An opening (see FIG. 7) may be formed on a rear side of the casing 10 allowing at least the controller 120 to pass through. Preferably, the opening is formed to allow even the heat pump module 110 and the steam generating module 130 to pass through.

According to an exemplary embodiment, the casing 10 may include a panel (not shown) closing the opening, and, in a preferable example, the panel is detachably coupled to the casing 10.

When the controller 120 is installed in the base 400 or detached from the base 400 for maintenance, the controller 120 may be transferred into or from the machinery room 120 through the opening. In particular, as shown in the example, in a case where the heat pump module 110 is disposed above the controller 120 and the blower 140 is disposed in front of the controller 120, the blower 140 or the controller 120 may not cause a problem when an attempt is made to detach the controller 120, so that the controller 120 may be drawn out from the machinery room 12 without detaching the blower 140 from the device assembly 2, and thus, it may be easy to repair or replace the controller 120.

The steam generating module 130 may be disposed inside the space S, and, preferably, above the controller 120. The steam generating module 130 may be fixed by the supporter 170. One of the steam generating module 130 and the supporter 170 may have a steam generating module fixing tab (not shown) formed thereon, the other one may have an installation hole 175 (see FIG. 5) into which the steam generating module fixing tab is inserted. The steam generating module installation part 175 may be formed on at least one of a pair of the supporters 170. Preferably, the installation hole 175 is formed on the supporter 170(1), but not limited thereto.

The steam generating module installation hole 175 may be formed on the side surface 173b of the shelf 173. With the steam generating module fixing tab being inserted into the steam generating module installation hole 175, the steam generating module 130 is temporarily installed in the supporter 170. Then, being temporarily installed in the supporter 170(1), the steam generating module 130 may be secured to the supporter 170(2) by a fastening member.

Hereinafter, referring to FIG. 4, there is a procedure of how to assemble the device assembly 2. In Fig. 4, a dotted line indicates a location and a direction in which a fastening member is secured. As shown in (a) of FIG. 4, when the compressor 160 is mounted on the base 400, the heat pump module 110 may be mounted on the supporter 170. The connection part 115 and 116 of the heat pump module 110 is fixed onto the shelf 173 by a fastening member. A refrigerant pipe which guides refrigerant may be connected between the compressor 160 and the heat pump module 110.

Referring to (b) of FIG. 4, the controller 120 may be inserted into the space S below the supporter 170 and mounted on the base 400. After the controller 120 is placed at a right position by inserting the controller fixing tab 121 into the controller fixing hole 414, the controller 120 and the base 400 may be secured by a fastening member.

Referring to (c) of FIG. 4, the blower 140 may be mounted in front of the supporter 170. The blower 140 may be secured to a blower installation tab 420 by a fastening member.

Referring to (d) of FIG. 4, a water drain pump 182 and a water supply pump 186 may be mounted on the base 400. One of the water drain pump 182 and the water supply pump 186 may be disposed on one side of the blower 140, and the other one may be disposed on the opposite side thereof.

Referring to (e) of FIG. 4, the air intake duct 150 may be mounted in front of the blower 140. A pin (not shown) extended in a front-and-rear direction may be formed on any one of the blower 140 and the air intake duct 150, and a pin hole (not shown) into which the pin is inserted may be formed on the other one. After the air intake duct 150 is placed in a right position due to insertion of the pin into the pin hole, the air intake duct 150 and the blower 140 may be coupled to each other by a fastening member.

Referring to (f) of FIG. 4, after the steam generating module 130 is inserted into the space S below the supporter 170, the steam generating module fixing tab formed on the steam generating module 130 is inserted into the installation hole 175, so that the steam generating module 130 may be placed at a right position and then the steam generating module 130 and the supporter 170 may be coupled to each other by a fastening member. The fastening member is preferably coupled to the supporter 170(2) so as to make it easy to detach the steam generating module 130 through an opening formed on the rear surface of the casing 10 for maintenance and repair.

Referring to (g) of FIG. 4, a noise filter 183 and a handle 184, which user holds to move the device assembly 2, may be mounted on the base 400. The noise filter 183 lets only necessary components in a signal input to the controller 120 to pass, and reduces noise components.

Referring to (h) of FIG. 4, the water supply hose 185 connecting the water supply pump 186 and the steam generating module 130, a nozzle module, a hose connecting the nozzle module 181 and a sump, a hose connecting the nozzle module 181 and the water drain pump 182, and any other various hoses may be connected to devices.

## Claims

1. A fabric treating apparatus comprising:
a casing (10) including a treating chamber (11) for clothes, and a machinery room (12) disposed below the treating chamber (11);
a base (400) defining a bottom of the machinery room (12);
a heat pump module (110) for performing air-conditioning on air supplied to the treating chamber (11);
at least one supporter (170) supporting the heat pump module (110) and limiting a predetermined space (S) between the at least one supporter (170) and the base (400) below the heat pump module (110);
a steam generating module (130) for generating steam to be supplied to the treating chamber (11); and
a controller (120) for controlling at least one of the heat pump module (110) and the steam generating module (130), wherein the heat pump module (110), the steam generating module (130), and the controller (120) are disposed in the machinery room (3), **characterized in that** the steam generating module (130) is mounted in the predetermined space (S) between the at least one supporter (170) and the base (400) below the heat pump module (110),
that the controller is disposed below the steam generating module (130) in the predetermined space (S), and that the supporter (170) comprises:
a pair of legs (171, 172) fixed on the base (400); and
a shelf (173) supported by the legs (171, 172),
wherein the heat pump module (110) is supported by the shelf (173).

2. The fabric treating apparatus of claim 1, wherein a supporter fixing tab (174) including a surface facing the base (400) is formed at a lower part of the leg, and
a supporter fixing part (410) including a supporter fastening hole (411) into which the supporter fixing tab (174) is inserted is formed in the base (400).

3. The fabric treating apparatus of claim 2, wherein the base (400) is made of a metal material, and the supporter fixing part (410) is formed by plastic working of the base (400).

4. The fabric treating apparatus of claim 3, wherein the supporter fixing part (410) is formed by cutting a portion having a predetermined length from the base (400) in a direction crossing a direction in which the supporter fixing tab (410) is inserted and then upwardly pressing a predetermined peripheral area of the cut portion.

5. The fabric treating apparatus of claim 4, wherein the supporter fastening hole (411) is formed due to plastic deformation of the cut portion.

6. The fabric treating apparatus of any one of claims 3 to 5, wherein the base (400) and the supporter (170) are coupled to each other by a fastening member which penetrates the supporter fixing part (410) and the supporter fixing tab (174) in a vertical direction.

7. The fabric treating apparatus of any one of claims 1 to 6, wherein, the casing (10) includes an opening formed at a rear side of the controller (120) to allow the controller to pass therethrough, the heat pump module (100) is disposed above the controller, and the fabric treating apparatus further comprises a blower (140) which transfers air into an interior of the heat pump module (110).

8. The fabric treating apparatus of claim 7, further comprising:
a panel (50) detachably coupled to the casing (10) and closing the opening of the machinery room (12).

9. The fabric treating apparatus of claim 8, wherein the blower (140) is disposed in front of the supporter (170).

10. The fabric treating apparatus of claim 8 or 9, wherein the blower (140) is coupled to the base (400).

11. The fabric treating apparatus of claim 10, further comprising:
an air intake duct guiding air flow introduced into the blower (140),
wherein the air intake duct is disposed in front of the blower (140).

12. The fabric treating apparatus of claim 11, wherein the air intake duct is fixed by at least one of the blower (140) and the base (400).

13. The fabric treating apparatus of claim 1, wherein a steam generating module fixing tab is formed at one of the steam generating module (130) and the supporter (170), and an installation hole into which the steam generating module fixing tab is inserted is formed at the other.

14. The fabric treating apparatus of claim 13, wherein the pair of supporters (170(1), 170(2)) is spaced apart from each other in a front and rear direction of the base (400), the steam generating module installation part is formed on one of the pair of the supporters (170(1), 170(2)), and
the steam generating module (130) is coupled to the other one of the pair of the supporters by a fastening member while the steam generating module fixing tab is inserted into the installation hole.

## Patentansprüche

1. Gewebebehandlungsvorrichtung mit:
einem Gehäuse (10), das eine Behandlungskammer (11) für Kleidung und einen Maschinenraum (12) aufweist, der unter der Behandlungskammer (11) angeordnet ist;
einer Grundplatte (400), die einen Boden des Maschinenraums (12) definiert;
einem Wärmepumpenmodul (110) zum Durchführen einer Klimatisierung an Luft, die der Behandlungskammer (11) zugeführt wird;
mindestens einen Träger (170), der das Wärmepumpenmodul (110) hält und einen vorgegebenen Raum (S) zwischen dem mindestens einen Träger (170) und der Grundplatte (400) unter dem Wärmepumpenmodul (110) begrenzt;
einem Dampferzeugungsmodul (130) zum Erzeugen von Dampf, der der Behandlungskammer (11) zugeführt werden soll; und
einer Steuereinrichtung (120) zum Steuern von mindestens einem des Wärmepumpenmoduls (110) und des Dampferzeugungsmoduls (130), wobei das Wärmepumpenmodul (110), das Dampferzeugungsmodul (130) und die Steuereinrichtung (120) im Maschinenraum (3) angeordnet sind,
**dadurch gekennzeichnet, dass**
das Dampferzeugungsmodul (130) im vorgegebenen Raum (S) zwischen dem mindestens einen Träger (170) und der Grundplatte (400) unter dem Wärmepumpenmodul (110) angebracht ist,
dass die Steuereinrichtung unter dem Dampferzeugungsmodul (130) im vorgegebenen Raum (S) angeordnet ist,
und dass der Träger (170) aufweist:
ein Paar Beine (171, 172), die an der Grundplatte (400) befestigt sind; und
eine Auflage (173), die durch die Beine (171, 172) gehalten wird,
wobei das Wärmepumpenmodul (110) durch die Auflage (173) gehalten wird.

2. Gewebebehandlungsvorrichtung nach Anspruch 1, wobei eine Trägerbefestigungszunge (174), die eine Oberfläche aufweist, die zur Grundplatte (400) weist, an einem unteren Teil des Beins ausgebildet ist, und
ein Trägerbefestigungsteil (410), das ein Trägerbefestigungsloch (411) aufweist, das in die Trägerbefestigungszunge (174) eingesetzt ist, in der Grundplatte (400) ausgebildet ist.

3. Gewebebehandlungsvorrichtung nach Anspruch 2, wobei die Grundplatte (400) aus einem Metallmaterial besteht und das Trägerbefestigungsteil (410) durch Umformen der Grundplatte (400) gebildet wird.

4. Gewebebehandlungsvorrichtung nach Anspruch 3, wobei das Trägerbefestigungsteil (410) durch Schneiden eines Abschnitts mit einer vorgegebenen Länge aus der Grundplatte (400) in eine Richtung, die eine Richtung kreuzt, in der die Trägerbefestigungszunge (410) eingesetzt wird, und dann durch Pressen eines vorgegebenen Umfangsbereichs des geschnittenen Abschnitts nach oben gebildet wird.

5. Gewebebehandlungsvorrichtung nach Anspruch 4, wobei das Trägerbefestigungsloch (411) infolge einer plastischen Verformung des geschnittenen Abschnitts gebildet wird.

6. Gewebebehandlungsvorrichtung nach einem der Ansprüche 3 bis 5, wobei die Grundplatte (400) und der Träger (170) durch ein Befestigungselement aneinander gekoppelt sind, das das Trägerbefestigungsteil (410) und die Trägerbefestigungszunge (174) in eine vertikale Richtung durchdringt.

7. Gewebebehandlungsvorrichtung nach einem der Ansprüche 1 bis 6, wobei das Gehäuse (10) eine Öffnung aufweist, die an einer Rückseite der Steuereinrichtung (120) ausgebildet ist, um die Steuereinrichtung dort hindurch gehen zu lassen, das Wärmepumpenmodul (100) über der Steuereinrichtung angeordnet ist und die Gewebebehandlungsvorrichtung ferner ein Gebläse (140) aufweist, das Luft in ein Inneres des Wärmepumpenmoduls (110) überführt.

8. Gewebebehandlungsvorrichtung nach Anspruch 7, die ferner aufweist:
eine Platte (50), die abnehmbar an das Gehäuse (10) gekoppelt ist und die Öffnung des Maschinenraums (12) verschließt.

9. Gewebebehandlungsvorrichtung nach Anspruch 8, wobei das Gebläse (140) vor dem Träger (170) angeordnet ist.

10. Gewebebehandlungsvorrichtung nach Anspruch 8 oder 9, wobei das Gebläse (140) mit der Grundplatte (400) gekoppelt ist.

11. Gewebebehandlungsvorrichtung nach Anspruch 10, die ferner aufweist:
einen Luftansaugkanal, der einen Luftstrom in das Gebläse (140) einleitet,
wobei der Luftansaugkanal vor dem Gebläse (140) angeordnet ist.

12. Gewebebehandlungsvorrichtung nach Anspruch 11, wobei der Luftansaugkanal durch das Gebläse (140) und/oder die Grundplatte (400) befestigt ist.

13. Gewebebehandlungsvorrichtung nach Anspruch 1, wobei eine Dampferzeugungsmodul-Befestigungszunge an dem Dampferzeugungsmodul (130) oder an dem Träger (170) ausgebildet ist, und ein Installationsloch, in das die Dampferzeugungsmodul-Befestigungszunge eingesetzt wird, am anderen ausgebildet ist.

14. Gewebebehandlungsvorrichtung nach Anspruch 13, wobei das Paar der Träger (170(1), 170(2)) in eine Vorwärts- und Rückwärtsrichtung der Grundplatte (400) voneinander beabstandet ist, das Dampferzeugungsmodul-Installationsteil an einem des Paars der Träger (170(1), 170(2)) ausgebildet ist, und
das Dampferzeugungsmodul (130) mit dem anderen des Paars der Träger durch ein Befestigungselement gekoppelt ist, während die Dampferzeugungsmodul-Befestigungszunge in das Installationsloch eingesetzt ist.

## Revendications

1. Appareil pour le traitement de tissu, comprenant :
une carrosserie (10) entourant un compartiment de traitement (11) pour des vêtements, et un compartiment technique (12) disposé sous le compartiment de traitement (11) ;
une base (400) définissant un fond du compartiment technique (12) ;
un module de pompe à chaleur (110) pour la climatisation de l'air amené dans le compartiment de traitement (11) ;
au moins un support (170) supportant le module de pompe à chaleur (110) et délimitant un espace défini (S) entre ledit au moins un support (170) et la base (400) sous le module de pompe à chaleur (110) ;
un module de génération de vapeur (130) destiné à générer de la vapeur à refouler vers le compartiment de traitement (11) ; et
un dispositif de commande (120) destiné à commander le module de pompe à chaleur (110) et/ou le module de génération de vapeur (130), le module de pompe à chaleur (110), le module de génération de vapeur (130) et le dispositif de commande (120) étant disposés dans le compartiment technique (3),
**caractérisé en ce que**
le module de génération de vapeur (130) est monté dans l'espace défini (S) entre ledit au moins un support (170) et la base (400) sous le module de pompe à chaleur (110),
**en ce que** le dispositif de commande est disposé sous le module de génération de vapeur (130) dans l'espace défini (S),
et **en ce que** le support (170) comprend :
une paire de pieds (171, 172) fixés sur la base (400) ; et
une tablette (173) supportée par les pieds (171, 172),
le module de pompe à chaleur (110) étant supporté par la tablette (173).

2. Appareil pour le traitement de tissu selon la revendication 1, où une cornière de fixation de support (174) présentant une surface opposée à la base (400) est formée sur une partie inférieure du pied, et où une partie de fixation de support (410) présentant un trou d'enclenchement de support (411) où est insérée la cornière de fixation de support (174) est formée dans la base (400).

3. Appareil pour le traitement de tissu selon la revendication 2, où la base (400) est en matériau métallique, et où la partie de fixation de support (410) est formée par usinage plastique de la base (400).

4. Appareil pour le traitement de tissu selon la revendication 3, où la partie de fixation de support (410) est formée par découpe d'une partie de longueur définie de la base (400) dans une direction croisant une direction d'insertion de la cornière de fixation de support (410), puis par emboutissage vers le haut d'une surface périphérique définie de la découpe.

5. Appareil pour le traitement de tissu selon la revendication 4, où le trou d'enclenchement de support (411) est formé par déformation plastique de la découpe.

6. Appareil pour le traitement de tissu selon l'une des revendications 3 à 5, où la base (400) et le support (170) sont raccordés par une élément de fixation s'engageant dans la partie de fixation de support (410) et la cornière de fixation de support (174) dans la direction verticale.

7. Appareil pour le traitement de tissu selon l'une des revendications 1 à 6, où la carrosserie (10) présente une ouverture formée sur un côté arrière du dispositif de commande (120) pour permettre le passage du dispositif de commande, où le module de pompe à chaleur (100) est disposé au-dessus du dispositif de commande, et où ledit appareil pour le traitement de tissu comprend en outre une soufflante (140) refoulant de l'air à l'intérieur du module de pompe à chaleur (110).

8. Appareil pour le traitement de tissu selon la revendication 7, comprenant en outre :
un panneau (50) monté de manière amovible sur la carrosserie (10) et fermant l'ouverture du compartiment technique (12).

9. Appareil pour le traitement de tissu selon la revendication 8, où la soufflante (140) est disposée en avant du support (170).

10. Appareil pour le traitement de tissu selon la revendication 8 ou la revendication 9, où la soufflante (140) est raccordée à la base (400).

11. Appareil pour le traitement de tissu selon la revendication 10, comprenant en outre :
un conduit d'admission d'air guidant un flux d'air passant dans la soufflante (140),
ledit conduit d'admission d'air étant disposé devant la soufflante (140).

12. Appareil pour le traitement de tissu selon la revendication 11, où le conduit d'admission d'air est fixé par la soufflante (140) et/ou par la base (400).

13. Appareil pour le traitement de tissu selon la revendication 1, où une cornière de fixation du module de génération de vapeur est formée sur le module de génération de vapeur (130) ou sur le support (170), et où un trou de montage où est insérée la cornière de fixation du module de génération de vapeur est formé sur l'autre élément - module ou support.

14. Appareil pour le traitement de tissu selon la revendication 13, où les supports de la paire (170(1), 170(2)) sont espacés l'un de l'autre vers l'avant et vers l'arrière de la base (400), où la partie de montage de module de génération de vapeur est formée sur un support de la paire (170(1), 170(2)), et où le module de génération de vapeur (130) est raccordé à l'autre support de la paire au moyen d'un élément de fixation, la cornière de fixation du module de génération de vapeur étant insérée dans le trou de montage.
